# EUROPEAN PATENT APPLICATION

(11) **EP 4 230 138 A1**
(43) Date of publication of application: **23.08.2023**
(21) Application number: 22305177.2
(22) Date of filing: 17.02.2022
(51) Int. Cl.: A61B 5/31, A61B 5/372, A61B 5/00

(54) **METHOD AND DEVICE FOR PHYSIOLOGICAL SIGNAL PREPROCESSING**

(71) Applicant: ICM (Institut du Cerveau et de la Moelle Épinière), 75013 Paris (FR); Institut National de la Santé et de la Recherche Médicale (INSERM), 75013 Paris (FR); Centre national de la recherche scientifique, 75016 Paris (FR); ASSISTANCE PUBLIQUE HOPITAUX DE PARIS, 75004 Paris (FR); SORBONNE UNIVERSITE, 75006 Paris (FR)
(72) Inventor: BURGUIERE, Eric, 75005 PARIS (FR); MONDRAGON GONZALEZ, Sirenia Lizbeth, 91310 LINAS (FR)
(74) Representative: Icosa

(57) **Abstract**

The present invention relates to a device and computer-implemented method for physiological signal preprocessing according to at least one biomarker to be detected in said physiological signal comprising: receiving discrete-time physiological signal and M filters, segmenting the at least one discrete-time physiological signal, computing a power spectral estimate of each subsegment, filtering each of said power spectral estimate with each filter of said M received filters to obtain a set of M coefficients; and defining, for each power spectral estimate, a feature vector comprising at least one coefficient of said set of M coefficients.

## Description

### FIELD OF INVENTION

The present invention relates to the field of signal processing, notably to the processing of physiological signals in view of the detection of at least one biomarker for the monitoring of the health of a subject, human and/or animal.

### BACKGROUND OF INVENTION

The analysis and modulation of physiological signals have become the basis of successful therapies and research programs in diverse fields. The objective monitoring and analysis of physiological signals have been used to feed and build main front-end health information systems remotely or in controlled environments (e.g., telemedicine and mobile health systems), and it has also been used to estimate emotional states (relevant in marketing and neuroscience). The modulation of physiological signals has proven to help alleviate the symptoms of treatment-resistant disorders. In the neurological and neuropsychiatric domains, invasive methods (such as deep brain stimulation and recording), and non-invasive methods of physiological monitoring (such as electrocardiogram, electromyogram, electroencephalogram, and magnetoencephalography) allow to quantitatively look at changes in the central and peripheral nervous system. It is then possible to make the causal link with the symptoms to monitor, to a specific behavioural response, or any other state relevant to the subject's wellbeing or the experiment. The analysis of physiological signals can give an essential insight into the subject's fluctuating internal and physical states and the evolution of the symptoms, as it allows objective measurements that are necessary for prompt and correct diagnosis and adjustment of treatment. However, such useful signals are often complex, non-stationary, with high-resolution, and multi-source, requiring multi-processing methods to reveal pertinent information.

Current methods for physiological signal-based recognition can be categorized into model-specific methods and unsupervised methods. The first one uses artificial intelligence (AI) that requires theory-driven features, meaning that observations or theories based on earlier knowledge direct the design of the feature's extraction. In this case, the features are usually interpretable, and there is usually a physical meaning allowing a further study on the model and the direct measures made. In clinical practice, there is usually a theory or an earlier observation of the subject state that justifies the monitoring of physiological signals in the first place. Given the properties and complexity of these signals, a dimensionality reduction stage or a feature optimization stage can significantly improve the performance of supervised approaches, and in some cases, it can also boost unsupervised methods. Indeed, too much irrelevant input data can lead to redundant features that take long to analyze and train the model. It can also lead to a space feature with a high dimensionality size problem causing a decrease in the model's performance. Different methods have been proposed to reduce dimensionality while keeping the primary information necessary for the subsequent analysis stages. One way to do this is by transforming the original features to a new reduced set of relevant and informative features that capture key characteristics of the signals.

Investigating the signals from the frequency domain can help reveal relevant characteristics of the physiological systems that otherwise would remain hidden in the time domain. For instance, many physiological signals have been demonstrated to carry different information across bandwidths. Therefore, changes in their properties (e.g., oscillations, gain, power spectral, etc.) can be related to changes in the underlying systems, as is regularly seen in neural, respiratory, vocal, inertial and cardiac signals. Nowadays, the classic frequency-domain approaches use combinations of band-pass filters to hard-narrow frequency bands. Given that usually physiological signals are sampled at medium to high sample frequency (tens to hundreds of Hz) depending on the nature of the signal, this rapidly scales up the amount of information to process.

Moreover, it is often the case that a particular state is related to changes in a specific frequency band. In this sense, signal biomarkers appear with a particular envelope in the frequency domain. However, current signal processing solutions often restrict the information by filtering between two cutoff frequency values without considering the complexity of a biomarker signature (i.e., unique characteristics that describe the biomarker, such as the power shift in a spectral band during a predefined period).

The present invention proposes a solution that allows for an efficient dimensionally reduction technique configured to extract the salient components of physiological signals that match a linked internal or external state. This approach discards all other information such as noise, reducing the data density and the computational cost. It also allows for simpler models used in the following signal analysis steps, enabling better human interpretation of the preprocessed signal.

### SUMMARY

This invention thus relates to a device for physiological signal preprocessing according to at least one biomarker to be detected in said physiological signal. Said device comprises:
- at least one input adapted to receive:
   - at least one discrete-time physiological signal previously acquired from at least one acquisition channel;
   - M filters comprised in a set of filters, wherein each filter of said set of filters has a predefined shape, comprised in a predefined frequency range, fa and fb, and wherein said set of filters has a distribution, defined by a predefined frequency scale, distributed around at least one principal frequency of interest F comprised in the range from fₐ to f_{b};
- at least one processor configured to:
   - segment the at least one discrete-time physiological signal, for each acquisition channel, using a predefined window so as to obtain overlapping discrete-time subsegments having a predefined time shift;
   - compute a power spectral estimate (i.e., a power spectrum) of each subsegment by transforming each subsegment from its discrete-time representation to a discrete-frequency representation;
   - filter each of said power spectral estimate with each filter of said M received filters to obtain a set of M coefficients;
   - defining, for each power spectral estimate, a feature vector comprising at least one coefficient of said set of M coefficients;
- at least one output adapted to provide said feature vector.

Advantageously, the present device (and method implemented by said device) is configured to transform the original features of the physiological signal in the discrete-time domain to a new reduced set of relevant and informative features that capture key characteristics of the signals. Indeed, the present invention method includes a coefficients decomposition stage that accurately represents the biomarker envelope by reducing acquired physiological signals to a few relevant filter sets' coefficients whose scale parameters vary with the biomarker envelope. The current approach does not restrict the information between two cutting frequency values, which is often the case with existing signal processing solutions. The precent method allows to drastically reduce the number of variables to work with, but it also provides an adapted frequency resolution that is centered to the targeted biomarker. By doing so, the following stages of signal processing (e.g., representation, classification, denoising) require less computing power and allow for simpler architectures. This effective procedure can be easily implementable in real-time configurations combined with AI methods to identify biomarkers in physiological signals. This method presents particular advantages when implemented as a preprocessing step for a physiological signal analysis pipeline to allow predicting symptom appearance merely based on the reading of neural signals.

According to other advantageous aspects of the invention, the device comprises one or more of the features described in the following embodiment, taken alone or in any possible combination.

According to one embodiment, the at least one processor is further configured, after filtering each of said power spectral estimate, to apply a decorrelation transformation to each set of M coefficients.

According to one embodiment, the at least one processor is further configured, after segmenting, to filter the overlapping subsegments for noise removal.

According to one embodiment, computing a discrete-frequency representation for each subsegment is performed using a spectral density estimation technique, such as discrete Fourier analysis, wavelet transform, the Multitaper method, Bartlett's, and Welch's method.

According to one embodiment, the predefined window length depends on the discrete-time physiological signal or the at least one biomarker to detect.

According to one embodiment, the predefined frequency range, the predefined frequency scale and principal frequency of interest F of the set of filters depend on the at least one biomarker to detect.

According to one embodiment, the at least one processor is further configured to, after decorrelation, normalize each set of M coefficients.

According to one embodiment, wherein the feature vector associated to one power spectral estimate comprises a coefficient being a function of said associated power spectral estimate.

According to one embodiment, the full wight half maximum of the at least one filter comprising the at least one principal frequency F is smaller than the full wight half maximum of the other filter in the set.

The present invention also relates to a method for physiological signal preprocessing according to at least one biomarker to be detected in said physiological signal, said method comprising:
- receiving:
   - at least one discrete-time physiological signal previously acquired from at least one acquisition channel;
   - M filters comprised in a set of filters, wherein each filter of said set has a predefined shape, comprised in a predefined frequency range, fa and fb, and wherein said set of filters has a distribution, defined by a predefined frequency scale, distributed around at least one principal frequency of interest F comprised in the range from fa to fb;

- segment the at least one discrete-time physiological signal, for each acquisition channel, using a predefined window so as to obtain overlapping discrete-time subsegments having a predefined time shift;
- compute a periodogram power spectral estimate of each subsegment by transforming each subsegment from its discrete-time representation to a discrete-frequency representation;
- filter each of said periodogram power spectral estimate with each filter of said M received filters to obtain a set of M coefficients;
- defining, for each periodogram power spectral estimate, a feature vector comprising at least one coefficient of said set of M coefficients;
- outputting said feature vector.

According to other advantageous aspects of the invention, the method comprises one or more of the features described in the following embodiment, taken alone or in any possible combination.

According to one embodiment, the method further comprises, after filtering each of said power spectral estimate, applying a decorrelation transformation to each set of M coefficients.

In addition, the disclosure relates to a computer program comprising software code adapted to perform a method for preprocessing a physiological signal compliant with any of the above execution modes when the program is executed by a processor.

The present disclosure further pertains to a non-transitory program storage device, readable by a computer, tangibly embodying a program of instructions executable by the computer to perform a method for preprocessing a physiological signal, compliant with the present disclosure.

Such a non-transitory program storage device can be, without limitation, an electronic, magnetic, optical, electromagnetic, infrared, or semiconductor device, or any suitable combination of the preceding. It is to be appreciated that the following while providing more specific examples, is merely an illustrative and not exhaustive listing as readily appreciated by one of ordinary skill in the art: a portable computer diskette, a hard disk, a ROM, an EPROM (Erasable Programmable ROM) or a Flash memory, a portable CD-ROM (Compact-Disc ROM).

### DEFINITIONS

In the present invention, the following terms have the following meanings:

The terms **"adapted"** and **"configured"** are used in the present disclosure as broadly encompassing initial configuration, later adaptation or complementation of the present device, or any combination thereof alike, whether effected through material or software means (including firmware).

The term **"processor"** should not be construed to be restricted to hardware capable of executing software and refers in a general way to a processing device, which can, for example, include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processorreadable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

The term **"filter"** refers to a function configured to remove some components or features from a signal.

The term **"shape"** of the filter refers to a general function, such as a gaussian function or a triangle/hat function, for arbitrary real constant.

### BRIEF DESCRIPTION OF THE DRAWINGS

**Figure 1** is a block diagram representing schematically a particular mode of a device for physiological signal preprocessing, compliant with the present disclosure;
**Figure 2** is a block diagram representing schematically a second particular mode of a device for physiological signal preprocessing, compliant with the present disclosure
**Figures 3(a) and 3(b)** illustrate two examples of different configurations of a set of filters according to the envelopes of the biomarkers targeted. Figure 3(a) illustrates a biomarker that is found around a certain F frequency for which a symmetrical distribution of filters is appropriated for its detection. In this example, M has been set to 7. The second example, in Figure 3(b), illustrates a biomarker that is found in the low-frequency band for which the chosen distribution of filter set is asymmetrical and M has been set equal to 4.
**Figure 4** is a flow chart showing successive steps executed with the device for predicting of figure 1;
**Figure 5** diagrammatically shows an apparatus integrating the functions of the device for segmenting of figure 1.
**Figure 6** is a flowchart illustrating the implementation of the invention as part of a routine designed to acquire physiological signals of a subject to provide real-time analysis and feedback to said subject.
**Figure 7** is a flowchart illustrating one example of the implementation of the invention as part of a routine designed to predict and interfere with a pathologic behaviour in a murine animal model of compulsive disorder uniquely by processing neural signals in real-time.

On the figures, the drawings are not to scale, and identical or similar elements are designated by the same references.

### ILLUSTRATIVE EMBODIMENTS

The present description illustrates the principles of the present disclosure. It will thus be appreciated that those skilled in the art will be able to devise various arrangements that, although not explicitly described or shown herein, embody the principles of the disclosure and are included within its scope.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Thus, for example, it will be appreciated by those skilled in the art that the block diagrams presented herein may represent conceptual views of illustrative circuitry embodying the principles of the disclosure. Similarly, it will be appreciated that any flow charts, flow diagrams, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

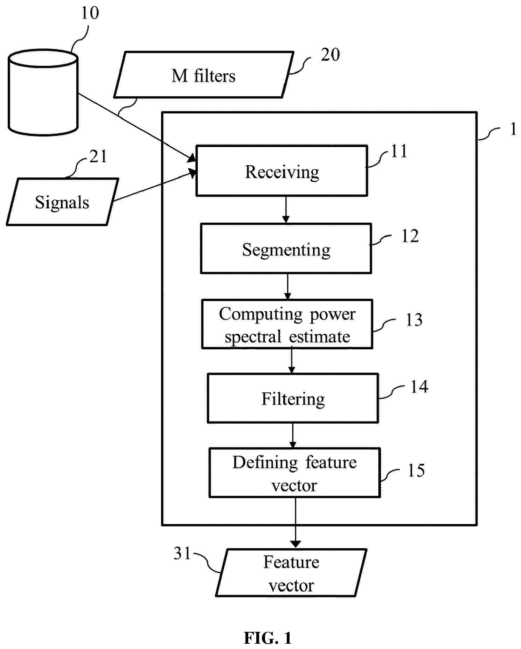

The present disclosure will be described in reference to a particular functional embodiment of a device 1 for physiological signal preprocessing, as illustrated on **Figure 1****.**

The device 1 is adapted to produce a matrix of coefficients that represents an energy distribution, associated to the envelope representative of the at least one biomarker to observe, in a time period during which the physiological signal had been analyzed. This matrix of coefficients represents a simpler structure than the initially acquired physiological signal, which may be more easily used to train or feed an artificial model designed to predict or detect specific changes in the physiological signals associated with the at least one biomarker (see the examples).

This physiological signal 21 may be neural, respiratory, vocal, motion and cardiac signals, which may be derived from a subject (i.e., healthy controls, patients, or in animal experimentation) through an invasive or non-invasive acquisition protocol (i.e., electrodes positioned on the surface of the skin or implanted). This physiological signal may be derived by a monitoring system configured to acquire and digitize signals sensed from N recording channels, with N going from 1 to a higher natural number depending on the type of monitoring system. An example of a monitoring system for deriving the physiological signal for the present invention may be an electrocardiogram device (EEG) configured to record neural activity from a subject's brain. The number of channels in an EEG system may range from as few as four electrodes to as many as 256 electrodes.

Other examples may be electrocardiogram systems, electromyogram systems and any other monitoring system configured to acquire a physiological signal known by the person skilled in the art. The physiological signals acquired are represented in the time domain, and since they are sampled signals, we will refer to the physiological signal also as time-discrete physiological signals.

Though the presently described device 1 is versatile and provides several functions that can be carried out alternatively or in any cumulative way, other implementations within the scope of the present disclosure include devices having only parts of the present functionalities.

The device 1 is advantageously an apparatus, or a physical part of an apparatus, designed, configured and/or adapted for performing the mentioned functions and produce the mentioned effects or results. In alternative implementations, the device 1 is embodied as a set of apparatus or physical parts of apparatus, whether grouped in a same machine or in different, possibly remote, machines. The device 1 may for example have functions distributed over a cloud infrastructure and be available to users as a cloud-based service, or have remote functions accessible through an API.

In what follows, the modules are to be understood as functional entities rather than material, physically distinct, components. They can consequently be embodied either as grouped together in a same tangible and concrete component, or distributed into several such components. Also, each of those modules is possibly itself shared between at least two physical components. In addition, the modules are implemented in hardware, software, firmware, or any mixed form thereof as well. They are preferably embodied within at least one processor of the device 1.

The device 1 comprises a module 11 for receiving the physiological signal 21, as well as the M filters 20 selected from the set of filters stored in one or more local or remote database(s) 10. The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk). In advantageous embodiments, the set of filters has been previously generated by a system including the device for generating a set of filters according to the properties of the biomarkers to observe. Alternatively, the M filters 20 from the set of filters are received from a communication network.

The set of filters may comprise T filters, wherein T is a natural number that may be comprised in the range going from 1 to 1000. The set of filters is defined so that each filter in the set is defined in a predefined frequency range, fa and fb, said boundary values fₐ and f_{b} being comprised in the frequency range (i.e., null value for filters or filters portions outside the predefined frequency range, fa and fb). The predefined frequency range depends from the at least one biomarker to detect. Indeed, the boundary points fₐ and fb are advantageously chosen to encompass a frequency range where the at least one biomarker is observed.

Moreover, each filter of said set of filters has a predefined shape. Notably all filters of the set have one same predefined shape. The filter shape may be symmetric such as for example a triangular shape or Gaussian shape or any other type of shape known by the person skilled in the art in the context of the present invention. The use of symmetric shape filters (i.e., triangular) that have a maximal response at the center frequency and decrease towards 0 until the adjacent center frequency of the neighbouring filters advantageously allows to have gradually decaying information concerning the portion of the spectrum to which they are applied.

Furthermore, the filter in said set of filters has a distribution, defined by a predefined frequency scale, distributed around at least one principal frequency of interest F. The principal frequency of interest F is comprised in the range from fₐ to f_{b}. The predefined frequency scale S(/) is defined in the range from fa to fb, which is the range approximating the signature of the at least one biomarker to detect. The frequency scale S(/) is advantageously chosen to mimic the shape and distribution of energy of the at least one biomarker signature in the frequency domain, notably by being more discriminative in the central frequency and less discriminative at frequencies where the energy decreases. The dynamic observed in the at least one biomarker signature could be mapped to standard distribution functions such as linear distribution, cumulative, or Gaussian distribution.

The filters in said set may have a symmetric distribution, defined by a predefined frequency scale, centered around one principal frequency of interest F. In the case of multimodal biomarkers having more than one principal frequency of interest F, the distribution of the set of filters may be defined so that filters are distributed around the multiple's principal frequencies of interest F. Furthermore, the filters in the set of filters may be all normalised so that their integral is equal to one.

Since the features extracted from the physiological signal depend in a great part on the distribution of the set of filters, the distribution function and its sampling can be suited to detect a variety of biomarkers for which a change in energy at certain frequency bands is observed.

In one example, the predefined frequency scale S(*f*) is linear. In this case, the distance between filters in the set is equally spaced, and the relationship between distance and center frequency (e.g., A/2 with A being the filter length) is linear. Alternatively, the predefined frequency scale S(/) representation could be a nonlinear function, such as a hyperbolic tangent function.

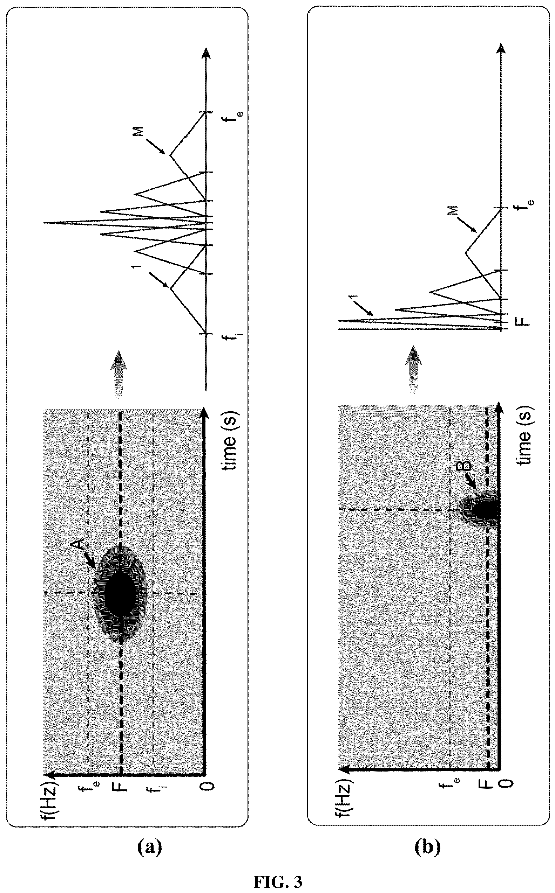

The set of filters may be defined (e.g., by appropriate choice of the shape and the scaling function) so that the full wight at half maximum of the at least one filter comprising the at least one principal frequency F is smaller than the full wight at half maximum of the other filter in the set. This feature advantageously allows to collect more granular information around the principal frequency F, which is the central frequency in a frequency band characteristic of the at least one biomarker to observe (see Fig. 3(a) and 3(b)). Furthermore, the set of filters is defined so that its filters are partially overlapped to avoid the risk of losing useful information during the application of the filters to the physiological signal.

According to one embodiment, the device for generating a set of filters according to the properties of the biomarkers to observe is configured to perform the following steps. A first step of receiving information/properties of the at least one biomarker signature to be observed. A second step of defining frequency range [*f*ₐ; *f*_{b}], as the boundary points comprise the at least one biomarker signature to be observed. A third step of defining a frequency scale S(*f*) between the defined frequency range [*f*ₐ; *f*_{b}], which approximates the signal signature to detect. Notably, said frequency scale S(/) is determined to obtain an optimal approximation of the signal signature's shape and energy distribution in the frequency domain. A fourth step of defining the set of filters with T filters f ∈ {1,2, ..., *T*} in the frequency range [*f*ₐ; *f*_{b}] using the frequency scale S(/). The frequency range [*f*ₐ; *f*_{b}], frequency scale S(/) and the number of filters T (i.e., parameters of steps 2, 3, and 4) can be determined with iterative testing routines in a validation step. Alternatively, these parameters may be chosen by a user and provided to said device for generating a set of filters.

According to one embodiment, the M filters received by the receiving module 11 are all the T filters of the set (i.e., M = T) or a selection of the T filters of the set (i.e., M inferior to T). The number T of filters of the set and M of the selected filters used will impact the number of features extracted. The extent of frequency bands of the signature to detect can be taken into account to decide the number of filters to use. A very sharp biomarker signature in frequency would probably require a smaller number than a biomarker signature that leaks over a large frequency band. The sampling frequency of the distribution function is chosen with respect to the number T of filters to be obtained for the set.

The device 1 further optionally comprises a module for standardization of the received physiological signals 21. The module may notably be adapted to the received physiological signals 21 for sake of efficient and reliable processing. This may enhance the efficiency of the downstream processing by the device 1. Such standardization may be beneficial when exploited signals originate from different sources, including possibly different signals acquisition systems.

The device 1 further comprises a module 12 for the segmentation of the incoming physiological signal 21 using a predefined time window. The length of the predefined time window may depend from the discrete-time physiological signal. Alternatively, the length of the predefined time window may depend on the at least one biomarker to detect, for example, the length of the signature of said at least one biomarker, the fundamental frequency suspected and/or the rapidity of changes in the signals.

The window size of the analysis window determines the frequency resolution, meaning how precise the analysis can be in frequency detection. Since the window size influences the frequency resolution of the analysis, the predefined window length may be set as a function of the frequency change pattern to detect. A narrow window will give a poor frequency resolution, and a wide window will have the opposite effect. Moreover, the predefined sampling frequency will determine the bandwidth's highest frequency we wish to detect.

As the present invention advantageously aims at detecting relevant changes in signals over time, the module 12 is configured to slide the predefined window on the at least one time-discrete signal 21 and to regularly apply it in order to define multiple time-discrete subsegments regularly overlapping with a predefined time shift (i.e., a distance equal to the predefined time shift between the starting time of the *i*^{th} segment and starting time of the *(i+1)^{th}* segment).

This predefined time shift depends mainly on three factors: (i) the computational speed that has to be high enough to allow real-time execution, (ii) how rapid are the fluctuations of the physiological signals 21, and (iii) the length of the predefined window.

In the case of the reception of physiological signals acquired simultaneously from multiple acquisition channels (e.g., electroencephalographic signals from a 10-20 system), the module 12 is configured to segment each physiological signal with the same predefined time window and the same predefined time shift.

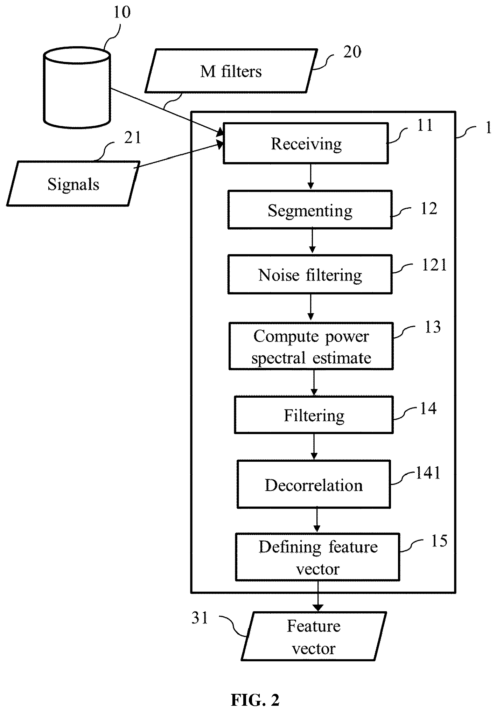

Optionally, the device 1 may comprise a denoise module 121, as shown in figure 2. The denoise module 121 is configured to apply at least one filter to the overlapping subsegments in order to reduce or remove the noise present. Indeed, physiological signals are often polluted with artifacts, thus, a processing step to eliminate external noise is required. The module 121 may be configured to filter for power line interference (removal of 50/60 Hz), removal of instrumentation and electronic artifacts which are usually found in the high-frequency bands, and when required, removal of motion artefacts (e.g., eye movement, blinks, and/or muscular artifacts generally detected in EEG signal) and/or respiratory movements. The denoise module 121 may be configured to apply filters such as notch filter, band-pass filter or any other filter or filtering method known by the person skilled in the art.

In the present invention, the feature decomposition stage proposes a transformation of time-discrete signal subsegments selected into analysis windows to discrete frames in the frequency domain that reflect a specific energy distribution over time around a frequency band of interest. The device 1 further comprises a module 13 for computing a power spectral estimate of each time-discrete subsegment obtained at the module 12. More in detail, the module 13 is configured to transform each time-discrete subsegment from its discrete-time representation to a discrete-frequency representation so as to obtain one power spectral (density) estimate for each time-discrete subsegment.

The discrete-frequency representation for each subsegment may be computed using a spectral density estimation technique. Different spectral density estimation techniques are suitable for the present invention such as the discrete Fourier transform, the wavelet transform, the Multitaper method, Bartlett's, or Welch's method.

The device 1 further comprises a module 14 for applying the M filters to a representation of the physiological signal. The module 14 is configured to apply each of the M filters received by the module 11 to each of the power spectral estimate obtained from module 13 which provides a coefficient for each of the M filter. Hence, this module 14 provides, as a result, a set of M coefficients for each power spectral estimate (i.e., each initial time-discrete subsegment). More in detail, applying one filter of the set to the power spectral estimate consists in the frequency domain to a multiplication between one filter and the power spectral estimate (i.e., frequency domain filtering). Advantageously, applying the M filters of the set allows decomposing the power spectral estimate capturing with high accuracy, small fluctuations around frequency bands of interest and the changes in the vicinity frequency bands with a lower resolution. This procedure allows to easily reduce the dimensional space to a few principal variables.

With reference to figure 2, the device 1 may further comprise a decorrelation module 141 configured to apply to each set of M coefficients a decorrelation transformation such as discrete cosine transformation, or principal component analysis and the like. Advantageously, this operation allows reducing cross-correlation between coefficients (i.e., caused by the fact that M filters are partially overlapping and therefore the resulting energies are correlated with each other). The decorrelation module 141 may be further configured to normalise each set of M coefficients to improve the signal-to-noise ratio.

The device 1 further comprises a module 15 for definition of a feature vector 31, destined to be used for biomarker detection. For each power spectral estimate (i.e., each time-discrete subsegment), this module provides a feature vector comprising at least one coefficient of said set of M coefficients. The feature vector 31 may include one or more coefficients that are function of the set of M coefficients or a parameter/feature calculated from the related power spectral estimate, such as its logarithm. The use of logarithm allows to enhance the low frequencies, for example when the signals to analyse are transient electrical signals generated in nervous (1-250 Hz) rather that single unit activity (350 -3kHz). Alternatively, all the M coefficients obtained by module 14 (decorrelated or not) are used as coefficients of the feature vector 31.

The present invention advantageously allows a feature extraction algorithm that is easy to implement, wherein only a few coefficients corresponding to the filtering collection are extracted from the physiological signals. Then the feature vector 31 may be used in a machine learning approach. Indeed, by strongly reducing the dimensionality of the feature space with representative variables of interest (i.e., M extracted coefficients), it is possible to train or feed a simpler structure in an artificial learning model. Multiple applicative examples are provided in the example section of the present application.

It may be observed that the operations by the modules 11, 12, 13, 14 and 15 are not necessarily successive in time and may overlap, proceed in parallel or alternate, in any appropriate manner. For example, new physiological signals may be progressively received over time and preprocessed, while the modules 12 to 15 are dealing with the previously obtained physiological signals. In alternative examples where the process of the present invention be performed offline, a batch of physiological signals 21 may be fully received and segmented before it is submitted to the modules 12 to 15.

The device 1 is further configured to output said feature vector 31 so that, for example, a data transfer module may retrieve the feature vector to use in a further processing pipeline. The device 1 may interact as well with a user interface, via which information can be entered and retrieved by a user. The user interface includes any means appropriate for entering or retrieving data, information or instructions, notably visual, tactile and/or audio capacities that can encompass any or several of the following means as well known by a person skilled in the art: a screen, a keyboard, a trackball, a touchpad, a touchscreen, a loudspeaker, a voice recognition system.

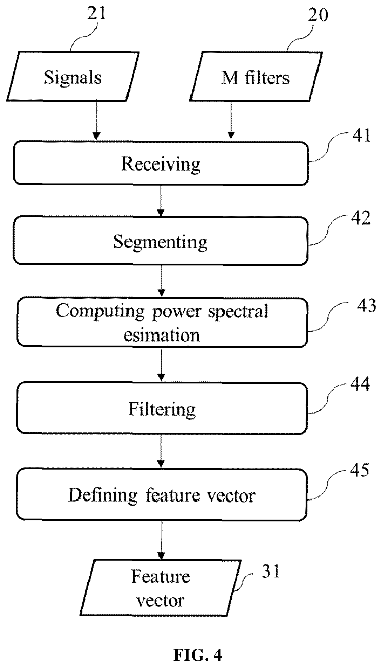

In its automatic actions, the device 1 may, for example, execute the following process (Figure 4):
- receiving the at least one physiological signal 21 and M filters 20 from a set of filters (step 41),
- segmenting the at least one discrete-time physiological signal for each acquisition channel, using a predefined window so as to obtain overlapping discrete-time sub segments having a predefined time shift (step 42),
- computing a power spectral estimate of each subsegment by transforming each subsegment from its discrete-time representation to a discrete-frequency representation (step 43),
- filtering each of said power spectral estimate with each filter of said M received filters to obtain a set of M coefficients (step 44),
- defining, for each power spectral estimate, a feature vector 31 comprising at least one coefficient of said set of M coefficients (step 45).

According to one embodiment, the process also comprises, after filtering each of said power spectral estimates (step 44), a step of applying a decorrelation transformation to each set of M coefficients.

According to one embodiment, the process also comprises, after segmenting (step 42), a step of filtering the overlapping subsegments for noise removal.

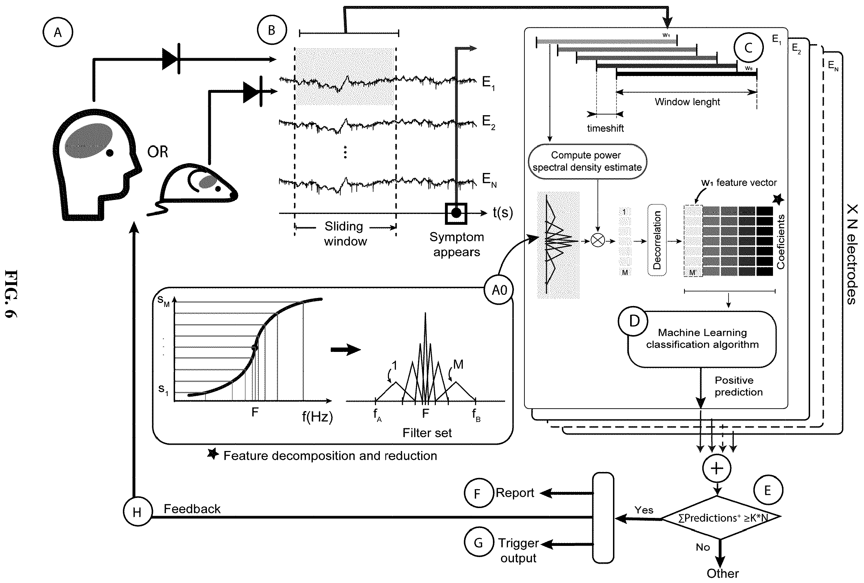

The steps of the process implemented by the device 1 described herein may be part of a preprocessing pipeline (steps B and C in Fig. 6) integrated into a more extensive routine for analysing physiological signal being implemented in real-time, as illustrated in Figure 6. Said routine that can be executed online or offline to monitor changes in the physiological signals, allowing a feedback or analysis system to react when at least one biomarker is detected. This detection causes one or various output triggers to be active, and their logical state can determine different outcomes. The routine may be configured so that the feedback consists of the activation of devices configured to interfere with the symptom's onset (i.e., symptoms associated with at least one detected biomarker). Such devices can be electrical stimulators, laser, optic stimulators, or any other visual, auditory, or sense stimulators that interact with the subject. In another example, the routine may be configured so that the feedback will activate a log entry in a registry. These registries can be used to: (i) monitor the subject's activity and symptom progression to diagnose, modify or assign treatment or therapies, (ii) supply feedback to the subject, (iii) modify the configuration of stimulators or external devices, (iv) collect individual information to be used in a further study (i.e., a populational or group studies), and/or (v) survey the effectiveness of a therapy or a drug in a clinical test. The goal is to record timepoints of valuable interest to the subject, clinicians, and/or researchers.

The intended use of the preprocessing device 1 of the present invention comes from the hypothesis that in any subject, its internal and external changes are correlated to changes in the physiological activity of reference and have, therefore, the potential to be monitored and exploited for the benefit of the subject. Biomarkers might or might not necessarily correlate with a subject's experience and sense of wellbeing, but they are objective and quantifiable characteristics of biological processes. Such changes can give rise to predictive or indicative physiological markers that can be observable in the frequency domain. An example of this is illustrated in Figure 3(a), where the appearance of an increment in the power of a particular band frequency, centered in F, correlates to the time point that precedes the expression or appearance of a symptom or event to detect.

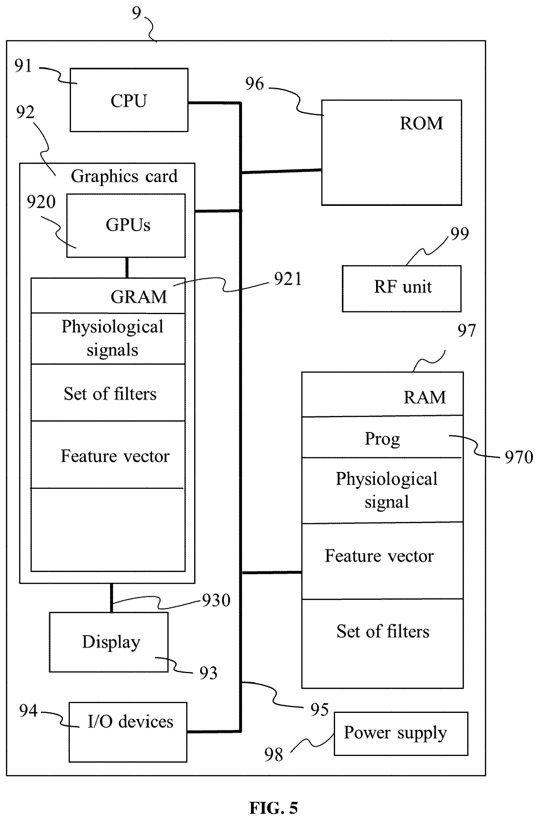

A particular apparatus 9, visible in Figure 5, embodies the device 1 described above. It corresponds, for example, to a workstation, a laptop, a tablet, a smartphone, programmable logical device (e.g., FPGA) for on-board calculation or a head-mounted display (HMD).

That apparatus 9 is suited to preprocessing physiological signals according to at least one biomarker to be detected. It comprises the following elements, connected to each other by a bus 95 of addresses and data that also transports a clock signal:
- a microprocessor 91 (or CPU);
- a graphics card 92 comprising several Graphical Processing Units (or GPUs) 920 and a Graphical Random Access Memory (GRAM) 921; the GPUs are quite suited to image processing due to their highly parallel structure;
- a non-volatile memory of ROM type 96;
- a RAM 97;
- one or several I/O (Input/Output) devices 94 such as for example a keyboard, a mouse, a trackball, a webcam; other modes for introduction of commands such as for example vocal recognition are also possible;
- a power source 98; and
- a radiofrequency unit 99.

According to a variant, the power supply 98 is external to the apparatus 9.

The apparatus 9 also comprises a display device 93 of display screen type directly connected to the graphics card 92 to display synthesized images calculated and composed in the graphics card. The use of a dedicated bus to connect the display device 93 to the graphics card 92 offers the advantage of having much greater data transmission bitrates and thus reducing the latency time for the displaying of images composed by the graphics card. According to a variant, a display device is external to apparatus 9 and is connected thereto by a cable or wirelessly for transmitting the display signals. The apparatus 9, for example, through the graphics card 92, comprises an interface for transmission or connection adapted to transmit a display signal to an external display means such as for example an LCD or plasma screen or a video-projector. In this respect, the RF unit 99 can be used for wireless transmissions.

It is noted that the word "register" used hereinafter in the description of memories 97 and 921 can designate in each of the memories mentioned, a memory zone of low capacity (some binary data) as well as a memory zone of large capacity (enabling a whole program to be stored or all or part of the data representative of data calculated or to be displayed). Also, the registers represented for the RAM 97 and the GRAM 921 can be arranged and constituted in any manner. Each of them does not necessarily correspond to adjacent memory locations and can be distributed otherwise (which covers notably the situation in which one register includes several smaller registers).

When switched-on, the microprocessor 91 loads and executes the instructions of the program contained in the RAM 97.

As will be understood by a skilled person, the presence of the graphics card 92 is not mandatory, and can be replaced with entire CPU processing and/or other implementations.

In variant modes, the apparatus 9 may include only the functionalities of the device 1. In addition, the device 1 may be implemented differently than a standalone software, and an apparatus or set of apparatus comprising only parts of the apparatus 9 may be exploited through an API call or via a cloud interface.

### EXAMPLES

The present invention is further illustrated by the following examples.

### Example 1: Stimulation and neuromodulation studies in an animal model of compulsive behaviour

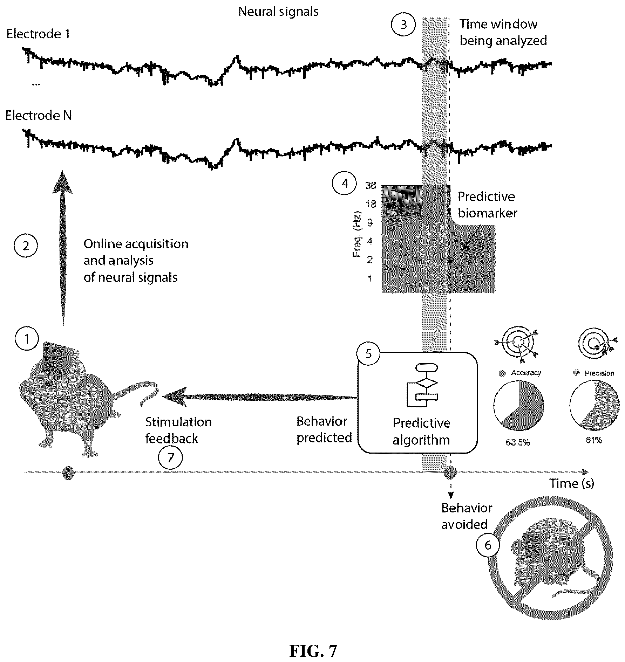

It was found that a non-sustained internally generated 1-4 Hz oscillation in the orbitofrontal cortex (OFC) temporally correlates with the onset of compulsive behaviour, which consists of exaggerated grooming behaviours, in an animal model of OCD (Fig. 7.4). To prevent compulsive behaviour, acute on-demand intervention is needed (Fig. 7.6 and 7.7). The invention described herein was implemented within our experimental framework to allow the automatic prediction of abnormal behaviour and the exploration of the underlying neuropathophysiology of compulsive behaviours. The prediction of the compulsive grooming behaviour is computed in real-time on a freely moving animal, and it is purely based on the continuous reading of neural signals.

The experimental framework that includes the invention put into practice is schematized in Fig. 7. Extracellular signals in the OFC are acquired through a chronic drive implant with 32 recording channels (Fig. 7.1). The physiological signals are acquired at a sampling frequency of 20 kHz, and they are amplified and digitalized while the animal can freely behave (Fig. 7.2). The analysis window length is predefined at 1 s with a predefined time shift of 200 ms (FIG 7.3). The filter set has a symmetrical configuration of seven triangular filters that follow a sigmoid distribution centred around 3 Hz. All seven filters of the set were used to calculate the coefficients that were further decorrelated performing the discrete cosine transformation. Five iterations were chosen to form a feature matrix of coefficients of size 5x7 (i.e., generation of one feature vector for iteration and use of the feature vectors as rows of the feature matrix).

According to this configuration, the features extracted were used to train a supervised classification algorithm with a minimal architecture: an artificial neural network with one input layer with 30 neurons, two hidden layers, and an output layer with two neurons, one for pre-compulsive behaviour and the other for a collection of different types of behaviour (Fig. 7.5). The database consists of electrophysiological signals of 1 s length corresponding to pre-compulsive behaviour events and other behaviours, they contain around 100 events per database. The databases were randomly split into a training data set containing 70% of the events and into a test data set containing the remaining 30% of events that were used to test the performance of the prediction algorithm. The decision was computed for each electrode separately; only if more than 80% of these detections pointed to a pre-grooming event, then it was counted as such. The invention described here advantageously allowed to use the local field potential signature observed to predict aberrant behaviour in a real-time experiment. The invention was put into practice for five different animals, for which the average performance at the output of the classification algorithm for a single recording channel was 61% precision and 64% accuracy. For the continuous processing of one second of 32 acquisition channels sampled at 20 kHz, the invention allowed reducing the input data to more than 99.99%. This protocol permitted real-time brain stimulation feedback that reduced the expression of compulsive behaviour by 62.37 % and its duration by 70.54%.

### Example 2: Predictive deep brain stimulation for preventing severe, chronic, treatment-refractory cases of psychiatric or movement disorders

Deep brain stimulation (DBS) has become the basis of successful therapies to alleviate the symptoms of severe treatment-refractory PD, OCD, hyperkinetic movement disorders and is currently investigated in other pathologies such as epilepsy and drug addiction. DBS stimulators provide continuous invariant stimulation at a fixed amplitude, frequency, and pulse width. The effectiveness of this open-loop therapy relies significantly on the determination of stimulation parameters by a clinician. Since stimulation parameters are programmed into the stimulator device and set to a constant setting until the next programming session, the approach does not take into account changes in the neural environment. Therefore, it is not sensitive to the changes or fluctuations of the disordered state. Without the necessary feedback, the device cannot adjust to alterations in the brain and is therefore limited in its use.

The possibility that pathological neural activity can be acquired directly from the target region using DBS electrodes has inspired a different type of paradigm: closed-loop adaptive DBS. Contrary to open-loop, a closed-loop DBS approach takes into account the alterations in the brain that reflect the state of the disorder and therefore decide to provide or not stimulation or choose to change the stimulation setting parameters. This strategy aims to identify pathological and physiological normal patterns of neural activity that can either help adapt stimulation parameters or deliver demand-dependent stimulation instead of continuous or random stimulation.

From a therapeutic point of view, there are many advantages to closed-loop DBS. First, chronic stimulation induces adverse effects of stimulation that could potentially be reduced with more precise short-duration stimulation. These effects are partially caused by unnecessary or excessive stimulation. Second, closed-loop DBS could reduce the amount of labour programming needed. Stimulation parameters are currently optimized through trial-and-error by a healthcare provider systematically changing variables such as pulse width, frequency, and amplitude to find a set of parameters that provide the best outcome. Indeed, programmers and clinicians confirmed that the device is a source of inconvenience since the tuning is far from optimal. It is time-intensive and observerdependent.

The few closed-loop DBS studies performed to date have significant limitations that slow down the transition from clinical trials to adopted cutting-edge therapies. In this context, the invention presented here aims to address the technology gap limitation:

First, it addresses the difficulty in creating and implementing control algorithms. Since the invention process complex signals and simplifies the information to a few features, it makes it a good candidate for the real-time analysis required in closed-loop DBS, where rapid reaction time is desired and non-redundancy and sufficiency in the inputs is primordial.

Second, since it uses simple mathematical operations and a reduced architecture, as shown in Fig. 3(a) it addresses the challenge of lightening up the computing power required from embedded implanted systems. In closed-loop DBS, sophisticated control systems with hundreds of processing operations or highly complex architectures, as those implemented in other artificial intelligence applications, might not be ideal since it is essential to bear in mind that this will be directly reflected in the battery power. In closed-loop DBS simple control algorithms that can extend the stimulator's battery life and reduce the number of surgeries needed for replacement are highly important.

## Claims

1. A device for physiological signal preprocessing according to at least one biomarker to be detected in said physiological signal, said device comprising:
- at least one input adapted to receive:
o at least one discrete-time physiological signal previously acquired from at least one acquisition channel;
o M filters comprised in a set of filters, wherein each filter of said set of filters has a predefined shape, comprised in a predefined frequency range, *f*ₐ to *f*_{b}, and wherein said set of filters has a distribution, defined by a predefined frequency scale, distributed around at least one principal frequency of interest F comprised in the range from *f*ₐ to *f*_{b};
- at least one processor configured to:
∘ segment the at least one discrete-time physiological signal, for each acquisition channel, using a predefined window so as to obtain overlapping discrete-time subsegments having a predefined time shift;
∘ compute a power spectral estimate of each subsegment by transforming each subsegment from its discrete-time representation to a discrete-frequency representation;
∘ filter each of said power spectral estimate with each filter of said M received filters to obtain a set of M coefficients;
∘ defining, for each power spectral estimate, a feature vector comprising at least one coefficient of said set of M coefficients;
- at least one output adapted to provide said feature vector.

2. The device according to claim **1,** wherein the at least one processor is further configured, after filtering each of said power spectral estimate, to apply a decorrelation transformation to each set of M coefficients.

3. The device according to either one of claims **1** or **2,** wherein the at least one processor is further configured, after segmenting, to filter the overlapping subsegments for noise removal.

4. The device according to any one of claims **1** to **3,** wherein computing a discrete-frequency representation for each subsegment is performed using a spectral density estimation technique, such as discrete Fourier analysis or wavelet transform.

5. The device according to any one of claims **1** to **4,** wherein the predefined window length depends on the discrete-time physiological signal or the at least one biomarker to detect.

6. The device according to any one of claims **1** to **5,** wherein the predefined frequency range, the predefined frequency scale and principal frequency of interest F of the set of filters depend on the at least one biomarker to detect.

7. The device according to any one of claims **2** to **6,** wherein the at least one processor is further configured to, after decorrelation, normalize each set of M coefficients.

8. The device according to any one of claims **1** to **7,** wherein the feature vector associated to one power spectral estimate comprises a coefficient being a function of said associated power spectral estimate.

9. The device according to any one of claims **1** to **8,** wherein the full wight half maximum of the at least one filter comprising the at least one principal frequency F is smaller than the full wight half maximum of the other filter in the set.

10. A method for physiological signal preprocessing according to at least one biomarker to be detected in said physiological signal, said method comprising:
- receiving:
∘ at least one discrete-time physiological signal previously acquired from at least one acquisition channel;
∘ M filters comprised in a set of filters, wherein each filter of said set has a predefined shape, comprised in a predefined frequency range, fa and fb, and wherein said set of filters has a distribution, defined by a predefined frequency scale, distributed around at least one principal frequency of interest F comprised in the range from *f*ₐ to *f*_{b};
- segmenting the at least one discrete-time physiological signal, for each acquisition channel, using a predefined window so as to obtain overlapping discrete-time subsegments having a predefined time shift;
- computing a power spectral estimate of each subsegment by transforming each subsegment from its discrete-time representation to a discrete-frequency representation;
- filtering each of said power spectral estimate with each filter of said M received filters to obtain a set of M coefficients;
- defining, for each power spectral estimate, a feature vector comprising at least one coefficient of said set of M coefficients;
- outputting said feature vector.

11. The method according to claim **10,** further comprising, after filtering each of said power spectral estimates, applying a decorrelation transformation to each set of M coefficients.

12. The method according to either one of claims **10** or **11,** wherein the predefined window length depends on the discrete-time physiological signal or the at least one biomarker to detect.

13. The method according to any one of claims **10** to **12,** wherein the feature vector associated to one power spectral estimate comprises a coefficient being function of said associated power spectral estimate.

14. A computer program product comprising instructions which, when the program is executed by a computer, cause the computer to carry out the method according to any one of claim **10** to **13.**

15. A computer-readable storage medium comprising instructions which, when executed by a computer, cause the computer to carry out the method according to any one of claim **10** to **13.**
